# EUROPEAN PATENT APPLICATION

(11) **EP 3 037 823 A1**
(43) Date of publication of application: **29.06.2016**
(21) Application number: 14199554.8
(22) Date of filing: 22.12.2014
(51) Int. Cl.: G01N 33/68

(54) **Method for determining pathological tissue damage and diagnosing infectious disease in fish**

(71) Applicant: BioMar Group A/S, 8000 Aarhus C (DK); Marine Harvest (Scotland) Limited, Rosyth, Fife KY11 2YW (GB); University Court Of The University Of Glasgow, Glasgow G12 8QQ (GB)
(72) Inventor: Eckersall, Peter David, 1QH Glasgow, G61 1QH (GB); Braceland, Mark James Thomas, Glasgow, G61 1QH (GB); Cockerill, David John, Fort William, PH33 7PT (GB); Tinsley, John William, Falkirk, FK3 8UL (GB); Bickerdike, Ralph Anthony, Falkirk, FK3 8UL (GB)
(74) Representative: Plougmann Vingtoft a/s

(57) **Abstract**

The present invention relates to methods for determining pathological tissue damage in fish. Also the present invention relates to methods for diagnosing infectious disease in fish.

In particular, the present invention is based on the surprising finding that mixing fish serum with an aqueous protein precipitation solution according to the present invention will result in a protein precipitation reaction if the fish is suffering from pathological tissue damage. Moreover it was surprisingly observed that the degree of turbidity of the precipitate in the mix of sample and aqueous protein precipitation solution was dependent on the state of infectious disease in fish.

## Description

### Technical field of the invention

The present invention relates to methods for determining pathological tissue damage in fish. Also the present invention relates to methods for diagnosing infectious disease in fish. In particular, the present invention is based on the surprising finding that mixing serum from fish with an aqueous protein precipitation solution according to the invention will result in a protein precipitation reaction if the fish is suffering from pathological tissue damage. Moreover the degree of turbidity of the precipitate from diseased fish serum is indicative of the severity of the pathological tissue damage and serial samples could be used to determine the progression of disease and pathology in fish.

### Background of the invention

Mortality and morbidity caused by infectious disease in e.g. Atlantic salmon, *Salmo salar,* remains one of the most challenging issues to the productivity and growth of aquaculture of the species. Reducing the effects of a disease outbreak relies on efficient diagnosis of disease for the implementation of disease management strategies. However, tools for cost effective regular monitoring of fish health are severely lacking in industry.

Aquaculture is the world's fastest growing livestock producing industry with an average annual growth rate of 3.2 percent being observed from 1961 to 2009 (FAO, 2012) and an estimated 154 million tonnes of fish being produced in 2011, overtaking production of wild catch fisheries. One of the most economically important finfish, in terms of North Western Europe in particular, is Atlantic salmon, *Salmo salar.* Aquaculture of this species alone has increased from 1990 to 2010 at an average annual rate exceeding 9.2% (FAO, 2012) and is projected to continue to rise to meet demand with a continually growing global population.

However, the salmon farming industry is currently facing a number of sustainability issues which may in the foreseeable future limit growth. These issues include (but are not limited to) fish meal and oil availability for feed (Tacon and Meitan, 2009) and suitable production sites (Asche et al 2013). In addition, in terms of its economic impact, one of the biggest challenges salmon aquaculture faces is that of infectious disease (Kibenge et al 2012).

Pancreas disease of Atlantic salmon (Salmo salar) which is caused by infection with salmonid alpha-virus (SAV) is associated with significant pathological damage to tissues. SAV infection leads to pancreatic pathology and marked myopathy affecting the heart and both red and white skeletal muscle. This is a major problem for the production of salmon by aquaculture.

Atlantic salmon aquaculture relies on the production of an end product which is profitable. In fish culture the main aim, therefore, is to produce flesh, comprised of skeletal muscle and fat, which is desirable in the market place. Quality of flesh is assessed in a number of ways (Larsson et al., 2012) and is the result of a number of complex genetic and nutritional processes. Upon harvest of fish the quality of flesh is the major determinant of price and thus profitability. Whilst quality downgrading of fillet can be caused by nutritional factors, toxic myopathies, and muscle breakdown due to excessive exertion (Larsson et al., 2012), one of the most important causes of skeletal muscle myopathy is infectious disease caused by viruses like (but not limited to) SAV.

Pancreas disease (PD) has been shown to have significant effects on fillet quality even after the fish is clinically healthy following recovery from the disease. This is most probably due to chronic effects of pathological tissue damage to skeletal muscle tissue and to pancreas damage resulting in reduced ability to uptake nutrients (Lerfall et al., 2011; Larsson et al., 2012). Whilst there are a number of non-destructive tools to detect its aetiological agent, salmonid alpha-virus (SAV), such as identifying presence of genes of the virus by reverse transcriptase polymerase chain reaction or established serology methods to identify antibodies specific to the virus in blood samples, there are not any tissue specific biomarkers currently available to diagnose and quantify associated pathologies. Therefore, a method for diagnosing pathological tissue damage, such as (but not limited to) skeletal muscle myopathy would have important application in the field of aquaculture, not only in diagnosing and quantifying the degree of PD-associated muscle myopathy but also pathological tissue damage caused by other diseases or stressors.

Despite various protein precipitation techniques being commonly used in the art in order to e.g. separate proteins from serum, the methods according to the present invention have hitherto not been disclosed in the art.

It is well-known that e.g. sodium acetate trihydrate is commonly used as a buffer in colorimetric assays for example in an assay for the blood protein ceruloplasmin based on the oxidation of paraphenylenediamine (PPD) substrate which is added to the buffer resulting in colour change being proportional to the amount of ceruloplasmin in a given sample. This methodology has been used in many investigations of mammalian species using sodium acetate trihydrate as buffer; however with no precipitation being reported. Similar methods have also been used in finfish species.

For example, Haluzova et al. (2010) demonstrated that ceruloplasmin activities were increasing after treatment of carps with the pesticide Sparatakus; however, again, with no precipitation of proteins being reported despite the presence of pathological damage in a number of the tested tissues. A similar buffer system has also been used with an acetate buffer and sodium azide solution mixture (i.e. with PPD substrate added) in Rainbow trout; however, again, no precipitate formation was observed (Yada et al. 2004).

Within the patent literature the following prior art should be mentioned.

WO2014/041189A1 discloses *inter alia* methods for treating, diagnosing, and tracking diseases associated with salmon alphavirus. WO2014/041189A1 also discloses methods for determining and/or identifying and/or quantifying a PD virus in a sample (e.g., a biological sample such as serum) using reagents containing oligonucleotides encoding the SPDV. There is however no disclosure in WO2014/041189A1 of precipitation reactions of serum for use in a method for determining/diagnosing skeletal muscle damage caused by e.g. PD in salmon.

EP 0 712 926 A2 discloses *inter alia* that fish pancreatic disease virus (FPCV) causes PD in Atlantic salmon and furthermore discloses methods of isolating FPCV, e.g. through cocultivation of infected tissues for the purpose of utilizing FPCV, or proteins or polypeptide derived therefrom as a PD-vaccine or in PD-diagnosis. Moreover, EP 0 712 926 A2 discloses salmon serum treated with e.g. phosphate buffers (e.g. PBS) for various types of antibody testing. There is however no disclosure in EP 0 712 926 A2 of precipitation reactions of serum for use in a method for determining/diagnosing skeletal muscle damage caused by e.g. PD in salmon.

US 4.171.204 discloses *inter alia* a method for analysing blood serum or plasma wherein the blood serum or plasma is treated with organic solvents such as acetonitrile, propionitrile, tetrahydrofuran or mixtures thereof in order to obtain a precipitate/supermatant of proteins which can be used in the examination for a component of interest. However, US 4.171.204 contains no disclosure of fish, salmon, fish diseases such as PD or other muscle tissue related diseases in fish. Moreover, there is no disclosure of precipitation reaction capable of diagnosing skeletal muscle damage caused by e.g. PD in salmon.

Recently a multi-biomarker test of serum from human patients to assess rheumatoid arthritis has been proposed (Centola et al., 2013). In this study, however, no acetate buffer, let alone sodium acetate trihydrate buffer, was used to precipitate proteins. In addition, precipitation reactions have been used in the prior art to identify specific antibodies in a biological sample (so-called precipitin tests) which indicate infection of with a given pathogen (Nielsen, 2002).

The use of biological markers to assess fish health can be thought of in two categories. The first of these can be markers of infection by aetiological agents of disease such as, acute phase proteins (Eckersall and Bell, 2010) where host immune response components are used to give an indication of whether there is an infectious agent present at a given time. These can be extremely useful as non-destructive health monitoring tools and have advantages over specific tests of virus presence and antibody presence commonly used in salmon aquaculture (Adams and Thompson, 2011) by being raised to detectable levels in fish serum much earlier than antibody. Additionally, agents such as acute phase proteins can also be used as markers when the aetiological agent is not known. However, it is widely established that aetiological agents can be present at a given site for a period of time before any clinical disease is observed or indeed any at all (Kristofferson et al., 2009; Jansen et al., 2010; Murray et al., 2011). This, in terms of disease management strategies, creates a problem as one cannot be certain when a clinical outbreak occurs, using the current diagnostic armoury available, unless histopathology assessment is routinely carried out which in turn reduces the output of fish to harvest and at significant sampling cost. As general biomarkers of pathological tissue damage, such as creatinine kinase, are being used ever more routinely in e.g. salmon aquaculture. Creatinine kinase assays have a number of limitations. For instance, the range of concentrations of creatine kinease in healthy sera due to a high and variable activity is large. This creates a problem making it hard to identify subtle changes in concentrations on a site to base health management decisions. Thus, more sensitive and tissue specific markers are needed for accurate diagnosis of clinical disease.

Hence, hitherto, the most well-established and reliable way of assessing myopathy in fish has been by histopathology. However, histopathology, compared to other means of assessment, is time consuming, costly, and requires a high level of expertise from the practitioner. In fact, histological assessment taken over a time course providing a measure of whether fish are at severe disease state, or whether they are in recovery from diseases such as PD, can take several months to fully resolve from initial infection.

However, despite these drawbacks, histopathology remains the gold standard for diagnosing myopathy in fish due to the limited number of reliable and sensitive alternative means of non-destructive health monitoring available. In light of this, there is a growing interest in identifying biomolecules that are capable of detecting/determining pathological tissue damage or infection using non-destructive techniques based on e.g. blood derived serum or plasma.

Hence, there is a need in the art of a simple and reliable method of diagnosing whether a fish is infected with microorganisms causing pathological tissue damage and/or a simple and reliable method to determining the state and severity of infectious diseases in fish.

### Summary of the invention

The present invention relates to methods for determining pathological tissue damage in fish as well as methods for diagnosing infectious disease in fish. In particular, the present invention is based on the surprising finding that mixing fish serum with an aqueous protein precipitation solution according to the present invention will result in a protein precipitation reaction if one or more tissues of the fish is/are pathologically damaged. Moreover, method of the present invention if based on the surprising observation that the amount of precipitate formed, e.g. determined as the degree of turbidity of the precipitate, was dependent on the state and severity of the infectious disease in fish.

Thus, the serum precipitation reaction of the present invention is based on fish tissue protein released into the blood following infection or trauma of the fish.

In particular, the present invention relates to methods of diagnosing pathological tissue damage in fish, such as pancreatic, cardiac or skeletal muscle tissue damage caused by e.g. pancreas disease (PD), in e.g. Atlantic salmon, *salmo salar.*

Hence, the method of the present invention could be used as a rapid and low cost test to indicate the presence and/or severity of infectious disease in fish. In addition, the method of the present invention could complement existing histological assessment and could be used as a way to quantify disease severity.

Since a common spectrophotometer and a low cost aqueous protein precipitation solution according to the present inventions can be used to measure the turbidity of the precipitate resulting from the method of the invention, the method could for example enable rapid analysis at fish farm locations.

Moreover, a rapid test kit could be easily and quickly developed by a pharmaceutical or diagnostics company to bring the invention to market. Thus, the method of the present invention could for example be used to provide documentation in the development or field validation of functional feeds or vaccines.

The precipitation reaction on which the present invention is based has e.g. been observed in serum of salmon with Pancreas Disease (PD), Heart and Skeletal Muscle Inflammation (HSMI) as well as Infectious Pancreatic Necrosis (IPN).

The method according to the invention relates to the hitherto unknown use of an aqueous protein precipitation solution for precipitating proteins from diseased fish serum in order to determine whether the fish is infected with pathogens that causes tissue damage. The inventors of the present invention have surprisingly found that the addition of serum from fish having pathological tissue damage, e.g. salmon infected with PD, results in a quantifiable precipitation of proteins which is differentially dependant on disease state/severity and the extent of pathological damage, to a number of tissues, such as skeletal muscle.

Thus, a first aspect of the present invention concerns a qualitative test which involves simple mixing of fish serum and an aqueous protein precipitation solution according to the present invention followed by visually observing, e.g. with the naked eye, whether the mixture becomes turbid or remains clear. No visible turbidity occurs in samples from clinically healthy fish.

Hence, in its simplest form; of observable turbidity upon addition of sera from fish with a severe pathological state of infectious disease, such as PD positive salmon, to the aqueous protein precipitation solution according to the present invention has promise as a quick, onsite and inexpensive point of care qualitative and quantitative test.

Moreover, although a slight precipitate formation can occur when applying the method of the present invention to healthy fish serum, it is not easily visible by the naked eye which, in turn, making the distinction between sera and tissue from healthy and diseased fish simple and effective.

Another aspect of the present invention relates to the quantitative monitoring of protein precipitation formation over a period of time, where differential precipitation over time has been shown to be an indicator not only of pathological damage to a given tissue but also to attain information on the extent/severity of the tissue damage, i.e. an indication of the stage of the disease.

Yet another aspect of the present invention is to provide a precipitate from the mixture of fish serum and an aqueous protein precipitation solution according to the present invention which subsequently after separation from the serum proteins remaining in solution, can be reconstituted in water followed by a protein separation step, e.g. by using electrophoresis, in order to obtain protein band profiles which, in turn, are indicative of what tissues are pathologically damaged.

In addition, the method according to the present invention may be used as an economic and rapid test for assessing the efficacy of protective/functional feeds, disease trial models and vaccines in fish health research.

More specifically, one aspect of the invention relates to a diagnostic method for determining whether a fish is infected with a pathogen capable of causing pathological tissue damage in a fish wherein the method comprises the consecutive steps of:
i) mixing fish blood serum with an aqueous protein precipitation solution that causes increased protein precipitation when admixed with serum from tissue damaged fish compared to the corresponding protein precipitation observed in clinically healthy fish,
ii) determining whether the fish is infected by establishing:
   iia) whether visible precipitate is formed, which in the affirmative is indicative of infection in the fish, or
   iib) whether the resulting mixture has no visible precipitate, which in the affirmative is indicative of clinically healthy fish.

Another aspect of the present invention relates to a diagnostic method for determining the stage of a pathogen causing disease resulting in pathological tissue damage in fish, the method comprising the consecutive steps of:
i) successively withdrawing blood samples from fish over a period of time (e.g. W0pc, W1pc, W2pc etc.)
ii) mixing the fish blood serum from the blood samples obtained under i) with an aqueous protein precipitation solution that causes increased protein precipitation when admixed with serum from tissue damaged fish compared to the corresponding protein precipitation observed in clinically healthy fish,
iii) determining the concentrations of the resulting protein precipitation samples from each of the mixtures obtained in step ii)
iv) comparing the concentrations of each of the protein precipitation samples and thereby determining the stage of the disease by establishing:
   iva) whether the precipitation concentration of a sample (e.g. W2pc) is higher than the concentration of the immediately preceding sample (e.g. W1pc) which in the affirmative is indicative of progress of disease, or
   ivb) whether the precipitation concentration of a sample (e.g. W2pc) is essentially equal to the concentration of the immediately preceding sample (e.g. W1pc) which in the affirmative is indicative of to steady state stage of the disease ivc) whether the precipitation concentration of a sample (e.g. W2pc) is lower than the concentration of the immediately preceding sample (e.g. W1pc) which in the affirmative is indicative of recovery from the disease.

Yet another aspect of the present invention is to provide a diagnostic method for determining the stage of a pathogen causing disease resulting in pathological tissue damage in fish, the method comprising the consecutive steps of:
i) successively withdrawing blood samples from fish over a period of time (e.g. W0pc, W1pc, W2pc etc.)
ii) mixing the fish blood serum from each of the blood samples obtained under i) with an aqueous protein precipitation solution that causes increased protein precipitation when admixed with serum from tissue damaged fish compared to the corresponding protein precipitation observed in clinically healthy fish,
iii) determining the optical density (level of turbidity) of the sample mixed with aqueous protein precipitation solution,
iv) determining the optical density (level of turbidity) in a sample mixed with aqueous protein precipitation solution (e.g. W2pc) and in the immediately preceding sample (e.g. W1pc)
v) optionally repeat step iv) with further successively obtained samples mixed with aqueous protein precipitation solution (e.g. W3pc, W4pc etc.),
vi) determining the change in optical density of the samples mixed with aqueous protein precipitation solution determined in step iv) and optionally in step v)
vii) thereby assessing the severity of the infectious disease as said severity is proportional with the degree of change in optical density (i.e. the higher the degree of change in optical density the more severe is the infectious disease).

### Brief description of the figures

Figure 1A: Optimization of precipitate assay.(A) Illustrates the effect of pH of buffer of delta OD of muscle lysate over a one hour period at 320nm absorbance wavelength. 0.2M sodium acetate trihydrate buffer was used for pH range 3.7 to 5.6 while 0.2M sodium phosphate buffer was used for pH 5.8 - 8.0.
Figure 1b: Optimization of precipitate assay. (B) Blank corrected optical density of resulting precipitation reaction over 60 minutes from the mixing of muscle lysate and buffer when using a range of different absorbance wavelength filters.
Figure 1c: Optimization of precipitate assay. (C) The kinetics of muscle lysate precipitation reaction when OD is monitored at either 340nm or 550nm absorbance wavelength.
Figure 1d: Optimization of precipitate assay. (D) Temperature effect on the formation of precipitate, monitored at 340nm absorbance wavelength using muscle lysate with OD recorded every second.
Figure 1e: Optimization of precipitate assay. (E) The effect of molarity on the delta OD at 340nm absorbance wavelength during a one hour time period when using W6pc sera and using sodium acetate trihydrate as protein precipitation solution.
Figure 2: Mean serum delta OD of precipitation reaction of serum from fish sampled at nine sample time points during a PD challenge. Bars represent mean +/- error bars as SEM using microtitre plate assay at 340 nm absorbance wavelength. Pool A is serum sample from clinically health salmon.
Figure 3: Comparison of severity of tissue pathology (mean histopathology score) in heart, read (slow) muscle pancreas or white (fast) muscle (as vertical histogram bars) with the mean delta change in turbidity in sera (straight line graph) according to week post challenge.
Figure 4: Coomassie blue stained 2DE separation of serum precipitate to compare protein profile at (A) W4pc serum (B) W0 serum. Lane 1 contains molecular weight markers. Stained bands highlighted in circles represent individual proteins that were differentially expressed in W4pc serum compared to W0 serum.

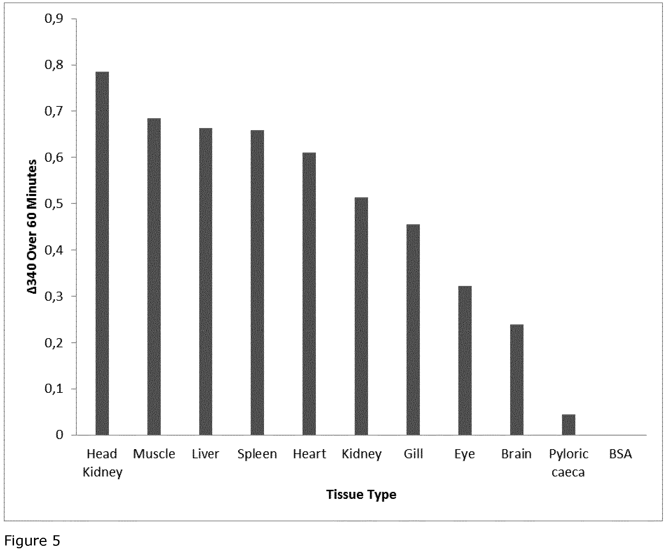

Figure 5: Protein precipitation from various organs of clinically healthy salmon tissue lysates. Bovine serum albumin (BSA) was used as a negative control. Bars represent mean delta OD at 340nm absorbance wavelength using spectrophotometer method.

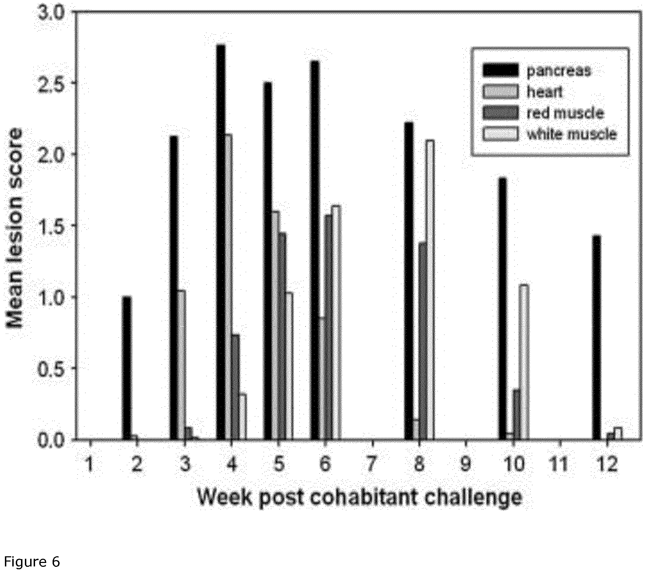

Figure 6. Time-course of pathology in pancreas, heart, red and white skeletal muscle in salmon with pancreas disease. Y-axis represents histopathology lesion scores from 0 - 3 as degree of pathology severity for each tissue where 0 represents healthy tissue and 3 is severe histopathology, according to a semi-quantitative scoring system (Table 1). X-axis is the time in weeks post challenge whereby fish directly infected with SAV were introduced to the population by cohabitation. Histogram bars represent mean histopathology score for pancreas, heart, red muscle then white muscle in the same sequence order for each time point post challenge. Where no bar exists represents mean histopathology lesion score of zero.
Figure 7: Monitoring of precipitate reaction using a range of aqueous buffers where salmon skeletal muscle lysate is mixed with the buffer/solution. Y-axis is optical density at 340nm absorbance wavelength. X-axis is time in minutes. SAT = Sodium acetate trihydrate, SC = Sodium citrate, SP = Sodium phosphate, PA = Potassium acetate, SS = Sodium sulphate, AP = Ammonium phosphate, MS = Magnesium sulphate, Blank = Water.
Figure 8: Monitoring of precipitate reaction using different concentrations of organic solvent where salmon skeletal muscle lysate is mixed with 10%, 50%, 75%, or 100% ethanol compared to blank with no ethanol. Y-axis is optical density at 340nm absorbance wavelength. X-axis is time in minutes.
Figure 9: Monitoring of precipitate reaction where salmon skeletal muscle lysate is mixed with aqueous ammonium sulphate (AS) at different ionic strengths; 0.2M, 0.5M, 0.75M, or 1M. Y-axis is optical density at 340nm absorbance wavelength. X-axis is time in minutes.
Figure 10: Monitoring of precipitate reaction where salmon skeletal muscle lysate is mixed with polyethylene glycol (PEG) at different molecular weights and concentration in Molarity. Y-axis is optical density at 340nm absorbance wavelength. X-axis is time in minutes. PEG1 represents 1,000Mw PEG and PEG2 represents an 8,000Mw agent, both at 0.01M or 0.05M concentration.
Figure 11: Differential precipitation in salmon serum of healthy (PLA) or with pancreas disease (W4) when using different aqueous buffers. The Y-axis is the difference in delta OD at 340nm absorbance wavelength for W4 compared to PLA sera for the same buffer. SAT = Sodium acetate trihydrate, SC = Sodium citrate, SP = Sodium phosphate, PA = Potassium acetate, SS = Sodium sulphate, Sca = Sodium Carbonate, AP = Ammonium phosphate, MS = Magnesium sulphate, AS 1M = Ammonium Sulphate 1M.
Figure 12: Difference in precipitation between salmon sera of healthy (PLA) or with pancreas disease (W4) using three different buffers over 1 hour. The Y-axis is optical density at 340nm absorbance wavelength. X-axis is time in minutes. Each line represents a different combination of sera and buffer where SAT = Sodium acetate trihydrate, SC = Sodium Citrate, PA = Potassium acetate.

The present invention will now be described in more detail in the following.

### Detailed description of the invention

The inventors of the present application surprisingly discovered that where serum of e.g. Atlantic salmon in a state of clinical Pancreas Disease (PD) was found to form a protein precipitate when mixed with sodium acetate trihydrate buffer. The resulting turbidity of the sample can be measured and quantified by for example spectrophotometry or other quantitative measuring techniques known in the art. Also surprisingly, the inventors of the present application observed that the extent of the turbidity correlated with the severity of e.g. PD related histopathology thereby providing an effective indicator of pathological tissue damage.

In addition, it has been observed that the precipitation reaction is much more pronounced when the fish, e.g. a salmon, is infected by a pathogen causing pathological tissue damage, e.g. SAV the causative agent of PD.

The precipitation reaction on which the present invention is based has also been observed in serum of salmon with e.g. clinical Heart and Skeletal Muscle Inflammation (HSMI) as well as Infectious Pancreatic Necrosis (IPN), which is not associated with skeletal muscle pathology, so the method of the invention is also applicable as a general indicator of severe pathological state due to infectious disease and/or immune reaction during acute and chronic diseases caused by viruses.

### The aqueous protein precipitation solution

In the context of the present invention, "precipitation solution" means an aqueous solution that causes increased protein precipitation when admixed with serum from diseased fish compared to healthy fish.

It has been demonstrated that a protein precipitation reaction occurs when serum from e.g. diseased Atlantic salmon is mixed with an aqueous protein precipitation solution according to the present invention.

It has been further demonstrated that the proteins which are precipitated in serum when combined with the precipitation solution originate from tissues as such they leak into the circulatory blood system as a result of pathological tissue damage. Lysates obtained from tissues have been used to characterise the precipitate reaction and method optimisation which therefore apply to the precipitation reaction in serum.

In certain embodiments of the method according to the invention,

Thus, in certain embodiments of the method according to the invention, the aqueous protein precipitation solution is chosen from solutions in water of one or more acids, bases, salts, organic compounds, inorganic compounds, ionic buffers or mixtures thereof.

In certain embodiments of the method according to the invention, the aqueous protein precipitation solution contains a salt chosen from ammonium sulphate, magnesium sulphate, ammonium phosphate, sodium carbonate, sodium sulphate, potassium acetate, sodium citrate, sodium phosphate, sodium acetate trihydrate or mixtures thereof.

In certain embodiments of the method according to the invention, the aqueous protein precipitation solution contains an organic compound chosen from methanol, ethanol, acetone or mixtures thereof.

In certain embodiments of the method according to the invention, the aqueous protein precipitation solution contains polyethyleneglycol (PEG).

### Ratios of serum and aqueous protein precipitation solution

In certain embodiments of the method according to the invention, the ratios between serum and aqueous protein precipitation solution is: 1: 10-20, 1: 11-19, 1:12-18, 1: 13-17, 1:14, 1:15, 1:16

In certain embodiments of the method according to the invention, when the aqueous protein precipitation solution is based on sodium acetate trihydrate, the ratio between serum and aqueous protein precipitation solution is: 1: 10-20, 1: 11-19, 1:12-18, 1: 13-17, 1:14, 1:15, 1:16 and most preferably 1:16 1/3 or 16 2/3.

### Heating of serum and aqueous protein precipitation solution before mixing

In certain embodiments of the method according to the invention, the serum is heated prior to the mixing with aqueous protein precipitation solution to: preferably, 20-40°C, more preferably 32-39°C, even more preferably 34-38°C and most preferably to 37°C.

In certain embodiments of the method according to the invention, the aqueous protein precipitation solution is heated prior to the mixing with serum to: 20-40°C, more preferably 32-39°C, even more preferably 34-38°C and most preferably to 37°C.

In certain embodiments of the method according to the invention, when the aqueous protein precipitation solution is based on sodium acetate trihydrate, the aqueous protein precipitation solution is heated prior to the mixing with serum to: preferably 20-40°C, more preferably 32-39°C, even more preferably 34-38°C and most preferably to 37°C.

### Molarity/ionic strength/strength of buffer in the aqueous protein precipitation solution

In certain embodiments of the method according to the invention, when the aqueous protein precipitation solution is based on sodium acetate trihydrate, the molarity of sodium acetate trihydrate buffer is between 0.05M -1M, more preferably between 0.1M - 1M, more preferably between 0.2M - 1M, more preferably between 0.3M - 1M, more preferably between 0.4M - 1M, more preferably between 0.5M - 1M, more preferably between 0.5M - 0.9M, more preferably between 0.5M - 0.8M, more preferably between 0.5M - 0.7M and most preferably 0.6 M.

In certain embodiments of the method according to the invention, when the aqueous protein precipitation solution is based on ammonium sulphate (AS), the ionic strengths of ammonium sulphate (AS) is between 0.2M - 1M, most preferably 0.2M.

In certain embodiments of the method according to the invention, when the aqueous protein precipitation solution is based on polyethylene glycol (PEG) where PEG1 represents 1,000Mw PEG, the ionic strengths of PEG1 is between 0.01M - 0.05M, most preferably 0.05M.

In certain embodiments of the method according to the invention, when the aqueous protein precipitation solution is based on polyethylene glycol (PEG) where PEG2 represents 8,000Mw PEG, the ionic strengths of PEG2 is between 0.01M - 0.05M.

In certain embodiments of the method according to the invention, when the aqueous protein precipitation solution is based on ethanol, the strengths of ethanol is between 10% - 100%, most preferably 10%.

### pH of the aqueous protein precipitation solution

In certain embodiments of the method according to the invention, the pH of the aqueous protein precipitation is preferably between 3.7 - 8.0, more preferably between 5.2 - 6.0, more preferably between 5.4 - 5.8, and most preferably 5.6.

In certain embodiments of the method according to the invention, when the aqueous protein precipitation solution is based on sodium acetate trihydrate, the pH of the solution is preferably between 3.7 - 5.6, more preferably 5, more preferably 5.2, more preferably 5.4, most preferably 5.6.

In certain embodiments of the method according to the invention, when solution is based on sodium phosphate, the pH of the aqueous protein precipitation is preferably between 5.6 - 8.0, more preferably 6.0, more preferably 5.8 and most preferably 5.6.

### The fish

In certain embodiments of the method according to the invention, the fish which is subjected to the diagnostic method(s) of the invention is of the family Salmonidae or may be of the subfamily Salmoninae. In a preferred embodiment, the fish may be of a genus selected from the group consisting of *Salmo, Oncorhynchus* and *Salvelinus.*

In certain embodiments of the method according to the invention, if the fish is of the genus *Salmo* the fish may be selected from the group consisting of Atlantic salmon *(Salmo salar* L.), Adriatic trout (*Salmo obtusirostris),* Flathead trout (*Salmo platycephalus),* Marble trout (*Salmo marmoratus),* Ohrid trout (*Salmo letnica),* Sevan trout (*Salmo ischchan)* and Brown trout (*Salmo trutta).*

In certain embodiments of the method according to the invention, if the fish is of the genus *Oncorhynchus,* the fish may be selected from the group consisting of rainbow trout (*Oncorhynchus mykiss),* coho salmon (*Oncorhynchus kisutch)* and Chinook salmon *(Oncorhynchus tshawytscha).*

In certain embodiments of the method according to the invention, if the fish is of the genus *Salvelinus,* the fish may be selected from the group consisting of Arctic char (Salvelinus alpinus).

In certain embodiments of the method according to the invention, the fish is selected from the group consisting of fry, fingerlings, parr, smolts and post-smolts.

In certain embodiments of the method according to the invention, if the fish is of the genus *Sparus,* the fish may be selected from the group consisting of gilt-head (sea) bream *(Sparus aurata).*

In certain embodiments of the method according to the invention, if the fish is of the family Serranidae, the fish may be selected from the genus Dicentrarchus of the group consisting of European seabass (*Dicentrarchus labrax),* and from the genus *Dicentrarchus* of the group consisting of Japanese seabass (*Lateolabrax japonicas*)*.*

In certain embodiments of the method according to the invention, if the fish is of the genus *Oreochromis,* the fish may be selected from the group consisting of Nile tilapia, *(Oreochromis niloticus).*

In certain embodiments of the method according to the invention, if the fish is of the genus *Pangasius,* the fish may be selected from the group consisting of Pangas catfish, (*Pangasius pangasius*)*,* and basa fish, (*Pangasius bocourti).*

In certain embodiments of the method according to the invention, if the fish is of the genus *Seriola,* the fish may be selected from the group consisting of Japanese amberjack or yellowtail, (*Seriola quinqueradiata*)*.*

In certain embodiments of the method according to the invention, if the fish is of the family Cyprinidae, the fish may be selected from the genus Ctenopharyngodon of the group consisting of grass carp (*Ctenopharyngodon idella*), the genus *Hypophthalmichthys* of the group consisting of silver carp (*Hypophthalmichthys molitrix)* and bighead carp (*Hypophthalmichthys nobilis),* the genus Cyprinus of the group consisting of common carp (*Cyprinus carpio),* the genus *Carassius* of the group consisting of crucian carp

(Carassius carassius), the genus *Catla* of the group consisting of major (Indian) carp (*Catla catla* or *Gibelion catla*), and the genus *Labeo* of the group consisting of Rohu or roho labeo (*Labeo rohita).*

### The pathogens

In certain embodiments of the method according to the invention, the pathogen causing tissue damage in the fish is chosen from the group of viruses consisting of but not limited to Salmon α-virus, salmonid alphavirus, Salmon Pancreas Disease Virus (SPDV), salmonid alphavirus (SAV), Piscine Myocarditis Virus, Piscine reovirus (PRV), Atlantic Salmon Calcivirus (ASCV), Aquabirnaviruses, Infectious pancreatic necrosis virus (IPNv), ISA virus or viral hemorrhagic septicemia virus (VHSV).

In certain embodiments of the method according to the invention, the pathogen causing tissue damage in the fish is chosen from the group of bacteria consisting of but not limited to *Aeromonas salmonicidia* subsp *salmonicidia, Vibrio salmonicidia, Vibrio anguillarum, Moritella viscosus, Piscirickettsia salmonis, Yersinia ruckeri* or *Flavobacterium psychrophilum.*

In certain embodiments of the method according to the invention, the pathogen causing tissue damage in the fish is chosen from the group of fungi consisting of but not limited to *Saprolegnia spp.*

In certain embodiments of the method according to the invention, the pathogen causing tissue damage in the fish is chosen from the group of parasites consisting of but not limited to *Neoparameabe peruans, Ichthyophthirius multifiliis.*

### The diseases

In certain embodiments of the method according to the invention, the disease that causes the pathological tissue damage in the fish is caused by one or more of the following virus diseases: Pancreas Disease (PD), Salmon Pancreas Disease (SPD), Heart and Skeletal Muscle Inflammation (HSMI), infectious pancreatic necrosis (IPN), Cardio Myopathy Syndrome (CMS), Infectious salmon anaemia (ISA), viral hemorrhagic septicemia (VHS), acute myopathy and immune reactions during chronic diseases caused by viruses.

In certain embodiments of the method according to the invention, the pathological tissue damage is caused by one or more of the following bacteria diseases: furunculosis, cold water vibriosis, vibriosis, winter ulcer disease, piscirickettsiosis, salmonid rickettsial septicemia (SRS), enteric redmouth (ERM), rainbow trout fry syndrome (RTFS), or bacterial kidney disease (BKD).

In certain embodiments of the method according to the invention, the pathological tissue damage is caused by one or more other acute or chronic stressors including fungi disease; Saprolegniasis, parasite diseases; Ameobic gill disease (AGD) or freshwater white spot disease.

### Intervals between withdrawal of blood sample

In certain embodiments of the method according to the invention, the withdrawal of blood samples form the fish are taken on an hourly basis, a daily basis, a weekly basis, a monthly basis or a yearly basis, most preferably on a weekly basis.

### Determining protein precipitation

In certain embodiments of the method according to the invention, the determination of visible precipitate is detected by the naked eye.

In certain embodiments of the method according to the invention, the optical density of precipitates formed on mixing sample with aqueous protein precipitation solution is determined by spectrometry using a wavelength between preferably 160-595nm and most preferably 340nm.

### Definitions

Prior to discussing the present invention in further details, the following terms and conventions will first be defined:
Δ_{340:} In the context of present application, any references to Δ₃₄₀ refers to the change in optical density (Δ₃₄₀) calculated by subtracting the first reading from the final for a specified time period. Said optical density refers to the reconstituted serum/buffer sample measured as the absorbance (Ab) measured at a wavelength 340nm by spectrophotometry in e.g. a Mictotitre plate reader or single cuvette instrument.
2DE: 2 dimensional electrophoresis.
Ab: absorbance as equivalent to optical density.

Buffer: A buffer is a solution containing either a weak acid and its salt or a weak base and its salt, which is resistant to changes in pH.

Clinically healthy salmon: in the context of the present application, clinically healthy salmon refers to clinical parameters are within a normal range and absence of infectious agent.
CMS: Cardiomyopathy syndrome.
CV: coefficient of variation (CV) is defined as the ratio of the standard deviation σ to the mean µ. It shows the extent of variability in relation to mean of the population.

Differential precipitation: the precipitation of one sample being not the same as another sample

Microtiter plate reader: In the present applications, any references to "ELISA plate reader" refers to laboratory instrument designed to detect biological, chemical or physical events of samples in small volumes in a microtiter plate, typically with a spectrophotometer.

Fish serum: In the context of present application, any references to "fish serum" refers to plasma component of blood with clotting factors removed.

Fish tissue lysate: In the context of present application, any references to "fish tissue lysate" refers to a fluid containing the contents of lysed cells from fish tissues.
HSMI: Heart and Skeletal Muscle Inflammation
IPN: Infectious Pancreatic Necrosis
MS/MS: a "tandem mass spectrometry" method for structure determination and analysis of molecules. Typically, MS/MS uses two mass spectrometers in tandem, wherein the two analyzers are separated by a collision gas cell.
Myopathy: Myopathy basically means muscle disease. A myopathy is a muscular disease in which the muscle fibers do not function for any one of many reasons, resulting in e.g. muscular weakness. In fish, skeletal muscle myopathies - causing muscle breakdown which in turn reduces the quality of the fish meat - are some of the most common symptoms of infectious diseases such as PD.
OD: optical density
Pathogen: in the context of the present application, pathogen refers to anything that can produce disease in a host. Pathogen may refer to a infectious agent such as a microorganism, virus, bacterium, prion, fungus or protozoan, that causes disease in its host.
Pathological damage: Predicted or actual progression of a particular disease in an organ, tissue or cell.
PD: Pancreas Disease.
Serum precipitation reaction (SPR): in the context of the present application, SPR is measured by change in absorbance over 60 minutes at 340nm
Pyloric caeca: in many fish, processed in finger-shaped pouches called pyloric caeca, which secrete digestive enzymes and absorb nutrients.
Salmonid: is a family of ray-finned fish, which includes salmon, trout, chars, freshwater whitefishes and graylings.
SAV: salmoinid alpha-virus.
SPR: serum precipitate reaction.
Turbidity: In the context of the present application, turbidity refers to the measure of relative clarity of a liquid solution.
Wpc: week post challenge with infectious microorganism (for example: W0pc refers to non-challenged fish - W4pc refers to fish, which have been challenged (infected for 4 weeks).

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Example 1 (obtaining serum samples)

Serum was collected from fish during the pathogenesis of PD from an experimental trial study described in Braceland et al (2013; 2014).

The feeding trial consisted of an acclimatisation phase and challenge phase which was carried out at Veso Vikan (Namsos, Norway). 360 Atlantic salmon parr of 30g with no previous contact with SAV were randomly distributed into duplicate 1m³ tanks and acclimated. Following acclimation, 120 fish were transferred from the acclimation tanks into triplicate 0.6 m³ tanks to be used for the disease challenge.

Naïve fish to be used as infective viral shedders (Trojans) were marked by clipping their adipose fin and injected with SAV 3 infected CHSE cell culture supernatant at ca. 10⁵ TCID/fish into their intraperitoneal cavity. Thirty inoculated Trojans were added to each of the challenge tanks 6 days after their assembly. Cohabitant fish serum was sampled at 0, 2, 3, 4, 5, 6, 8, 10 and 12 weeks post challenge. At each time point 9 fish per tank were killed by lethal overdose of anaesthetic benzocaine and blood collected in non-heparinised sample tubes from the caudal vein.

From 6 of the sampled fish, pyloric caecae and pancreas (hereafter referred to as pancreas), heart and skeletal muscle tissue were processed from standardised locations for histology. Tissues were routinely processed and stained with hematoxylin and eosin. Tissue samples were examined as a blind study by a pathologist and the scoring system used to semi-quantify the distribution and severity of the tissue lesions is described by McLoughlin et al. (2006) (Table 1).

**Table 1. Semi-quantitative lesion score system used to PD compare lesion severity in Atlantic salmon. (a) Pancreatic lesion classification. (b) Heart lesion classification. (c) Red and white skeletal muscle lesion classification.**

| Score | Description |
|---|---|
| (a) | |
| 0 | Normal appearance |
| 1 | Focal pancreatic acinar cell necrosis |
| 2 | Significant multifocal necrosis/atrophy of pancreatic acinar tissue, some normal tissue remaining |
| 3 | Total absence of pancreatic acinar tissue |
| R | Recovery of pancreas |
| (b) | |
| 0 | Normal appearance |
| 1 | Focal myocardial degeneration ± inflammation (<50 fibres affected) |
| 2 | Multifocal myocardial degeneration ± inflammation (50-100 fibres affected) |
| 3 | Severe diffuse myocardial degeneration ± inflammation (>100 fibres affected) |
| R | Repair and/or regeneration |
| (c) | |
| 0 | Normal appearance |
| 1 | Focal myocytic degeneration ± inflammation |
| 2 | Multifocal myocytic degeneration ± inflammation |
| 3 | Severe diffuse myocytic degeneration ± inflammation |
| R | Repair and/or regeneration |

### Example 2 (obtaining cell lysates of tissue samples)

Tissue samples were collected from clinically healthy fish from a commercial production fish farm site with no previous history of disease. The tissue lysates were e.g. compared to see which protein from different organs were similar to those observed in the serum protein precipitation reaction of infected fish.

Tissue samples were immediately stored on dry ice and subsequently frozen at -80°C until processing. Lysates were created using mortar and pestle technique known in the art, where tissue pieces were ground while kept frozen by periodic addition of liquid nitrogen until a fine powder formed. This was then transferred to another mortar and 10ml of cell lysis buffer (20mM Tris-HCL, pH 7.5) per 1g of tissue was added prior to further grinding and mixing for 5 minutes. The grinded tissue was then centrifuged at 13,000rpm at 4°C for ten minutes whereafter the removed supernatant was transferred into another tube and the centrifugation step was repeated. Solid residue was discarded and the supernatant was passed through a 0.45µm filter twice before storage at -80°C.

### Example 3 (the Serum Precipitation Reaction (SPR) assay)

### Plate reader

For use on a microtitre plate; 15µl of serum is dispensed to a 96 well plate and placed inside a microtitre plate reader (Fluorostar Optima BMG Labtech) equilibrated to 37°C. Sodium acetate trihydrate buffer (0.6 M at a pH of 5.6) was also heated to 37°C before addition of 245µl to each well. Absorbance (Ab) was measured with the plate reader at 340nm over a 60-minute duration with readings being taken every 30 seconds for each well to give optical density values at each time point. The change in optical density (Δ₃₄₀) in each sample was calculated by subtracting the first reading from the final for each.

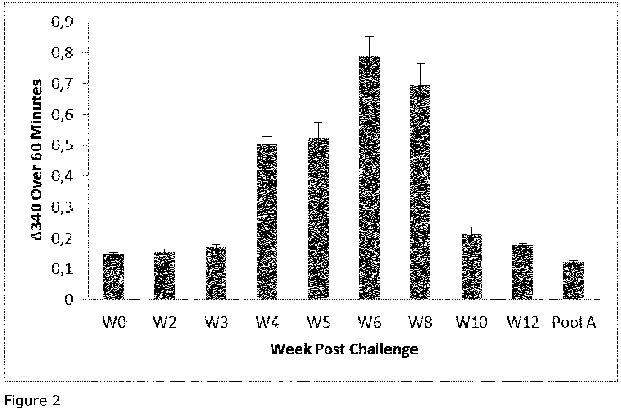

The results are shown in figure 2.

### Spetrophotometer

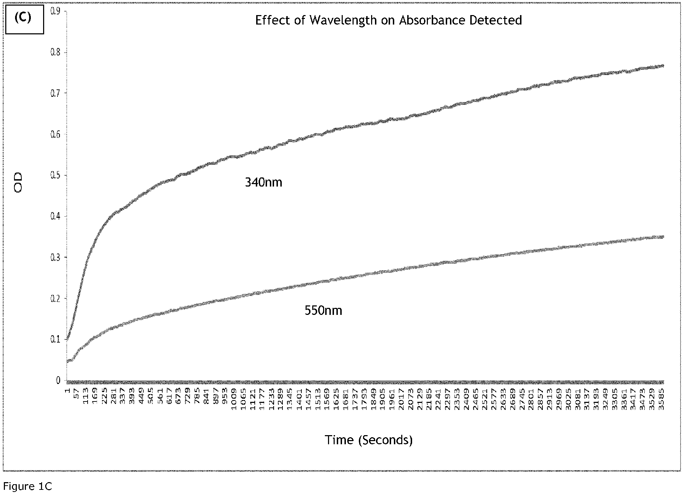

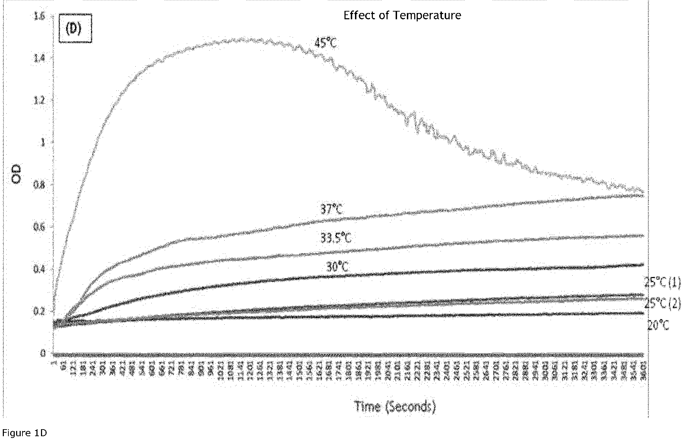

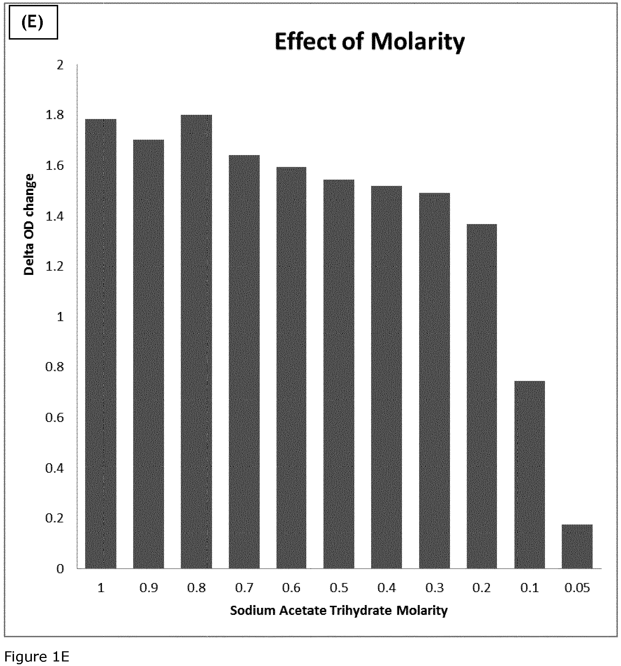

The precipitate assay was performed on a spectrophotometer (Jencons 7315) as follows. An aliquot of 180µl 2% (w/v) bovine serum albumin (BSA) was added to 60µl of serum sample before mixing in a cuvette. 1 ml of sodium acetate trihydrate buffer (0.6 M at a pH 5.6 was added to the cuvette. The BSA was shown not to have any effect on the reaction kinetics or precipitation reaction but reduced the sedimentation of precipitate allowing for more reliable OD to be monitored in the spectrophotometer test as by adding viscosity the sedimentation process takes longer, thus keeping precipitate suspended in the solution during the test allowing for more accurate readings. Monitoring of the OD was carried out at 340nm with readings taken every second for 60 min. Results are shown in Figures 1C, 1D, and 1E.

### Example 4 (SPR to determine changes in precipitation over time in fish infected with PD)

The SPR method according to the present invention was applied on individual samples to investigate the changes in precipitation over time and the relationship between degree of precipitation and pathological tissue damage caused by PD.

A pool of sera from clinically healthy fish (pool A) and mean serum precipitation reaction response of fish at week 4 post challenge (W4pc) in a severe pathological state were used to calculate interassay %CV, which were 10.6% and 9.7% respectively (n = 25 plates), average intra assay %CV was 7.2%.

Figure 2 shows SPR measured by the mean Δ₃₄₀ of serum from fish according to week post challenge (Wpc).

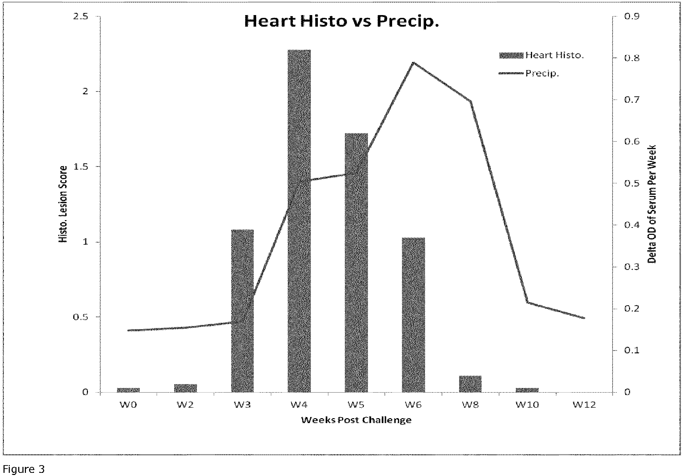

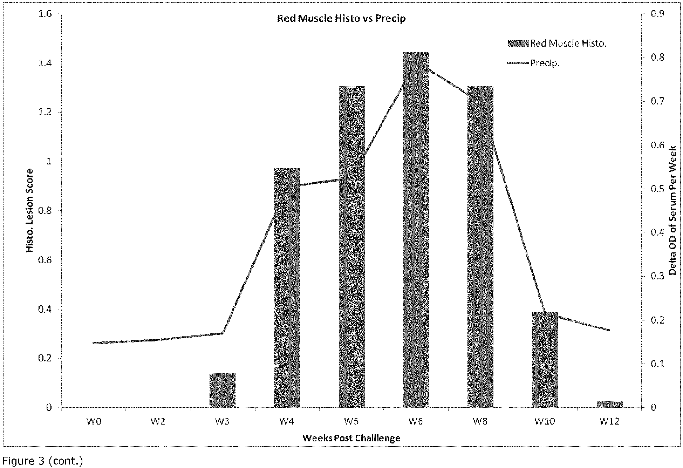

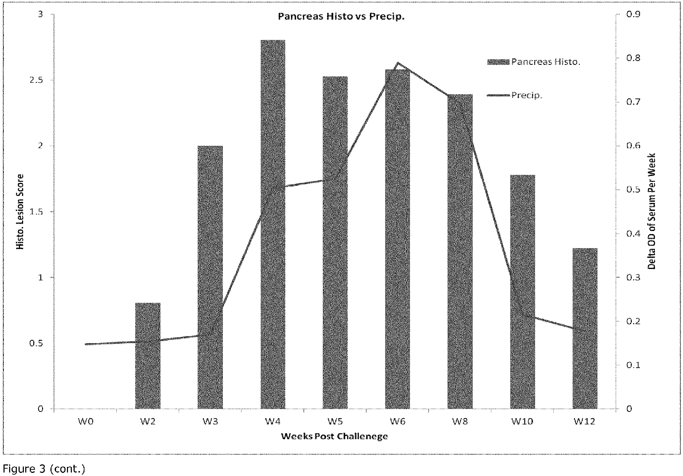

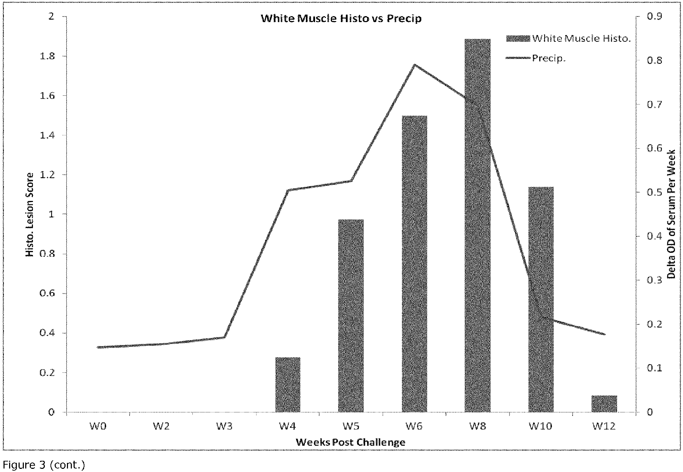

Thus, by using the method of the present invention the precipitation potential of individual samples was assessed with Δ₃₄₀ from 0 to 60 minutes. As is evident from figure 2, there were significant differences in SPR during the trial as the disease progresses with a significant rise in precipitation in sera at W4pc, before its peak at W6pc and returning to homeostasis near levels at W12pc. Also observed was a significant correlation between the pathological tissue damage and SPR (figure 3) which makes the SPR assay of the present invention applicable as a diagnostic test in the field as the test requires little specialised reagent and with a visible end-point could be easily performed at fish production sites. Figure 3 shows the four tissues affected by pancreas disease and the mean lesion scores during the study compared to the mean precipitate/ delta OD change during the progression of pancreas disease pathology.

These surprising findings makes the method a simple and effective tool for determining the severity of pathological tissue damage in fish over time, which can be of importance when monitoring of the health status of fish in aquaculture.

The invention has potential to improve fish health management decisions when a disease outbreak occurs in a fish farming facility, which in turn will lead to lower health related losses of the production unit. Specifically this invention will provide information on the timing of critical period of severe pathology and the degree of severity.

This knowledge can be used to implement mitigation strategies to minimise mortality and decrease the negative impact on farmed animal growth and feed utilisation caused by the disease pathology, thereby reducing the economic impact of the disease. These mitigation strategies may include reducing feeding rate, increased biosecurity measures and avoiding handling or movement of fish during the critical pathology period. Decreasing the number of destructive samples that are required will reduce fish health management costs and reduce the number of farmed animals to be culled thereby improving fish welfare standards of the farming industry.

This invention can also be used as a method for developing and assessing the efficacy of functional feeds, immune modulators or stimulants, medicines, disease trial models and vaccines by providing information and documentation on the severity of pathology in both the laboratory development of the products and for in the field validation and product support. The invention may reduce the number of animals required to be culled for biological tissue samples in these studies. Greater knowledge of disease processes within populations may also be obtained as results of the ability to sample from individual fish for epidemiology studies.

### Example 5 (SPR to determine pathological tissue damage in different fish tissues)

The inventors of the present application have thus observed that pathological damage to the tissues depicted in figures 3 and 6 would, as in the case of PD, cause a change in serum proteome constitution, that would increase the SPR and thus be applied as a general health test as a non-destructive tool for diagnosing clinical disease. Moreover, the ability for precipitate to be reconstituted in water and electrophoresis separation of proteins contained means there is the potential for identifying specific band profiles depending on disease/ pathology.

This observation was further validated by the significant correlation of Δ₃₄₀ to histopathology of all tested tissues by using Pearson correlation coefficient (as previously published in Braceland et al 2013) thus indicating the precipitate potential of a given serum sample may be influenced by multiple pathologies which could be valuable as a biomarker of damage to tissue in general.

Also, the precipitation reaction based on the Δ₃₄₀ using the Microtitre plate assay was found to be significantly correlated with histopathological data as described in Braceland et al. 2013 by Pearson coefficient analysis, with Δ₃₄₀ correlating positively with severity of histopathology in heart (p = 0.001), pancreas (p = <0.001), red muscle (p = <0.001), and white muscle (p = <0.001), thus indicating that pathological damage to all of the examined tissues had a significant effect on the precipitate potential of serum.

### Example 6 (optimization of the precipitation assay)

The quantifiable difference of turbidity depending on health status of individual fish providing the sera has lead to the development of a SPR based method of the present invention based upon Δ₃₄₀.

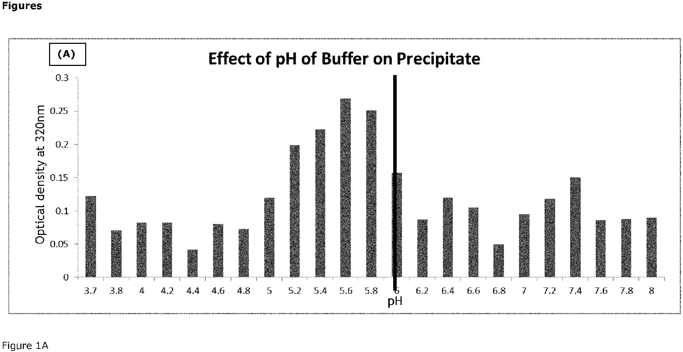

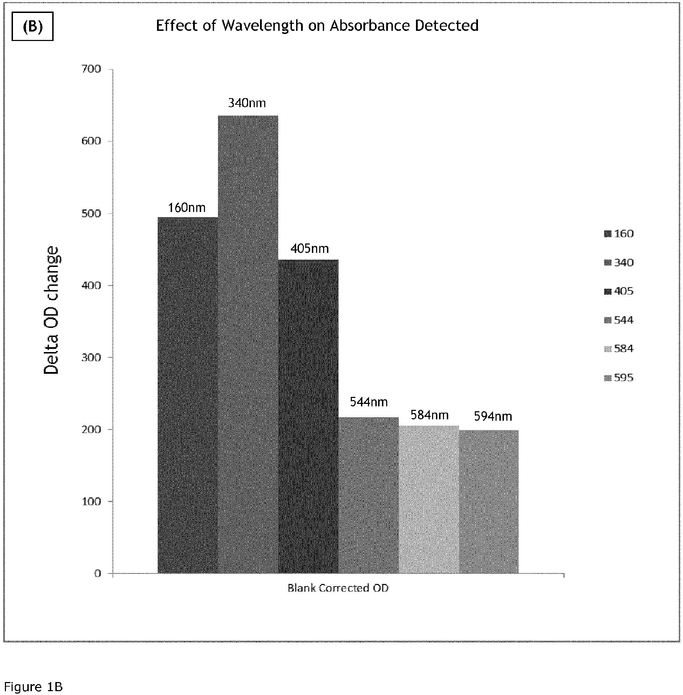

Through development and optimization it was found that this turbidity phenomenon is effected by a number of parameters, such as; temperature, and the molarity pH of the buffer used. For instance, precipitation formation is much faster at higher temperatures (figure 1d) and also more precipitate is formed over a given time when using a buffer of higher molarity (figure 1e) or pH of buffer (figure 1a). In addition, forming precipitate which manifests as turbidity is detected differently dependent on the wavelengths used (figure 1b and 1c).

Thus, a range of buffers with the desired pH were prepared by titrating sodium acetate (0.2M) with acetic acid (0.2M) and di-sodium hydrogen phosphate (0.2M) with sodium dihydrogen phosphate-2-hydrate to achieve a range from pH 3.7 to pH 8.0. Results indicated that a sodium acetate buffer with pH 5.6 was optimal for maximizing Δ₃₄₀ (figure 1a).

The optimal wavelength for determining changing turbidity caused by precipitation was examined by assessing a range of fixed wavelength detection filters to establish the optimal wavelength for observing precipitate formation by Microtitre plate reader with 340 nm yielding the greatest OD reading (figure 1b). This wavelength also gave higher absorbance using a spectrophotometer when compared to 550nm (figure 1c), confirming that a wavelength of 340nm was optimal.

Figure 1d shows the effect of temperature on the spectrophotometer assay. Increasing the temperature from 20°C to 37°C caused a steady increase in the absorbance at 340nm for the precipitation reaction. At 45°C there was a rapid increase followed by a fall in absorbance due to a sedimentation effect with precipitate settling out of the solution.

The effect of buffer molarity on precipitation carried out using a plate reader with 60µl muscle lysate being added to 200µl buffer. Figure 1e illustrates the wide range of molarity where precipitate is formed, although a significant drop in Δ₃₄₀ being observed when sodium acetate buffers of <0.2 M were used. From these results, the optimal assay conditions for the serum precipitate reaction (SPR) were determined to be: 0.6M sodium acetate trihydrate buffer at pH 5.6, and reaction temperature at 37°C.

Other factors, such as, buffer to sample ratio were also optimized and was found to be 15µl sample with 245µl buffer for analysis on Microtitre plate reader.

The optimal buffer to sample ratio for the spectrophotometer assay was found to be 1ml of buffer being added to 240µl of sample mix (the sample mix consisting of 60µl sample and 180µl 2% BSA) with changing absorbance being monitored over a 60 minute period (Δ₃₄₀).

### Example 7 (identification of the precipitated proteins)

### 2 Dimensional Electrophoresis (2DE) of precipitate

In order to investigate the cause of the serum precipitation reaction (SPR), W0pc and W4pc sera was mixed with sodium acetate buffer and after extensive washing was examined after reconstitution in water by using 2 dimensional electrophoresis (2DE) examination.

More specifically, to investigate the composition of the proteins forming the precipitate, separate W0pc and W4pc pools were created for examination with equal volumes from each time point. Samples of the serum pools (300µl) were added to 0.6M sodium acetate trihydrate buffer (1ml) and allowed to incubate for one hour in a water bath at 37°C. The solution was then centrifuged at 3000rpm for 10 minutes and supernatant collected. Precipitate was washed by re-suspending in 1ml of SAT buffer and mixed prior to spinning at 3000rpm. This was repeated twice and each time supernatants removed, the final time the precipitate was re-suspended in 200µl of distilled water and mixed until proteins had gone back into solution.

One microlitre of each serum sample collected from each fish sampled at each time point was pooled according to week to create pooled samples for the analysis of changing protein composition throughout the time course. The protein concentration of the pooled samples was determined by Bradford assay, using Bradford Reagent.

The Bradford protein assay is used to measure the total protein concentration of an aqueous sample of unknown protein content. The theory behind the method is that proteins bind to a Coomassie dye under acid conditions which results in a colorimetric change from brown to blue which can be measured quantitatively by spectrophotometry. The intensity of the blue colorimetric change is dependent on protein concentration and can be compared to a standard curve of known protein concentration, such as with BSA as standard. An equal protein loading for 2DE protein separation by isoelectric focusing based on isoelectric point (pI) and sodium dodecyl sulphate poly- acrylamide gel electrophoresis (SDS-PAGE) based on molecular weight. Three replicate 2DE gels were run of the pool of samples from each time point. Separation by pI was carried out using 11 cm immobilized pH Gradient (IPG) strip with a pH range of 3 to10 (BioRad, Hemel Hempstead, UK). After protein loading of the IPG strips with serum diluted in a rehydration buffer (8 M Urea, 2% CHAPS, 50 mM DTT, 0.2% Bio-Lyte®) (BioRad, Hemel Hempstead, UK) and covered in 500 µl of mineral oil, a combined rehydration and focusing step was carried out over 17 h with a total of 35,000 V-h. The IPG strips were removed, oil drained and then treated with two equilibration buffers both made from a stock solution comprised of 6 M urea, 0.375 M Tris-HCl, pH 8.8, 2% (w/v) SDS, 20% (v/v) glycerol, the first of these containing 2% (w/v) dithiothreitol (Sigma-Aldrich, Poole, UK) to reduce the proteins and subsequently the alkylating agent iodoacetamide at 2.5% (w/v) (Sigma-Aldrich, Poole, UK). IPG strips were then placed onto Criterion SDS-PAGE gels and submerged in XT Mops running buffer and subjected to electrophoresis at 200 V for one hour (Bio-Rad, Hemel Hempstead, UK). Subsequently gels were stained in Coomassie brilliant blue G-250 dye 0.1% (w/v) in destain solution for 1 h and then de-stained using a solution of methanol:water:acetic acid, (4:5:1) overnight, scanned and saved in 16-bit grey TIFF format images for gel image analysis.

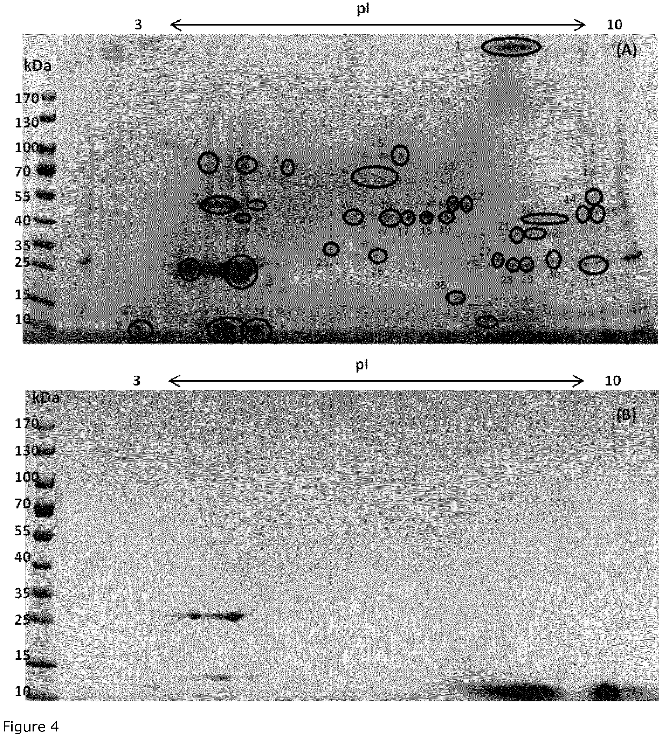

As can be seen from figure 4, the 2DE gel profile of precipitate formed from PD diseased (W4pc) serum is much more complex and diverse than that of the W0pc serum. The main difference in proteome that causes this differential precipitation is an increase in proteins (spot 6-22, 27-31, 35) that are usually intracellular being passively increased in the serum due to pathological tissue damage during PD.

For example, the detection of multiple isoforms of enzymes, such as CK (CK1, CK2, and CK3) indicates that the diagnostic method according to the present invention is not restricted to identifying pathologies in one specific tissue.

### Example 8 (SPR in muscle lysate)

The use of tissue lysates in this experiment was to determine whether proteins found in the serum precipitation reaction were tissue specific.

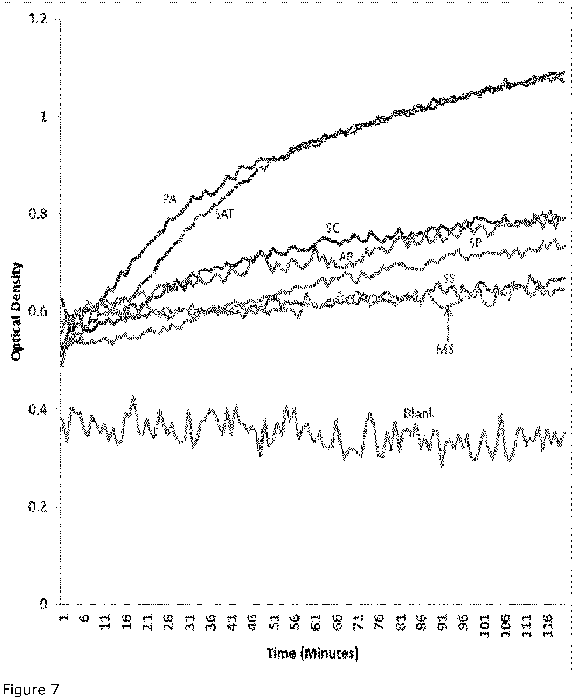

Moreover, through the introduction of muscle lysate to a number of different buffer types it has been shown that precipitation reaction is not limited to sodium acetate trihydrate (Figure 7).

All of these buffers were used at an ionic strength of 0,6M which was found when using sodium acetate trihydrate buffer (Figure 7) to cause a high level of precipitation. While all of these buffers seemed to also cause a precipitation reaction sodium acetate trihydrate (SAT) or Potassium acetate (PA) had the greatest effect. However, each buffer may have a greater precipitation effect at different molarities.

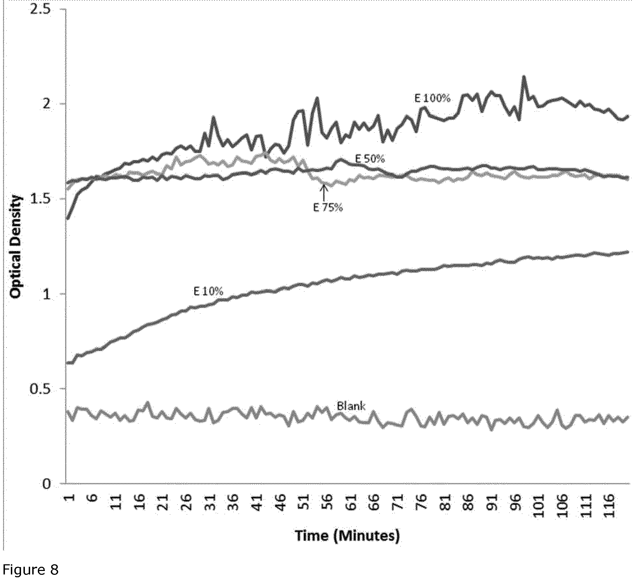

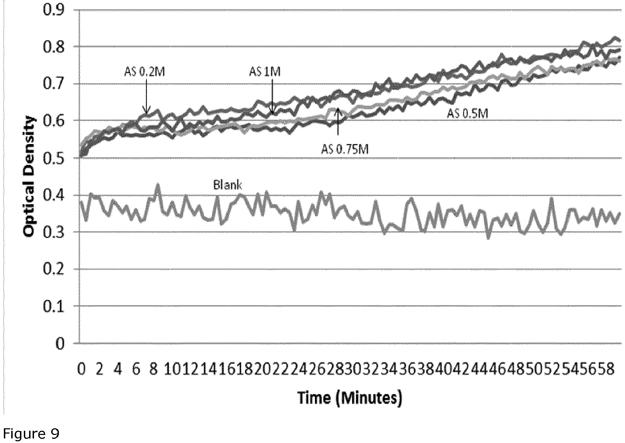

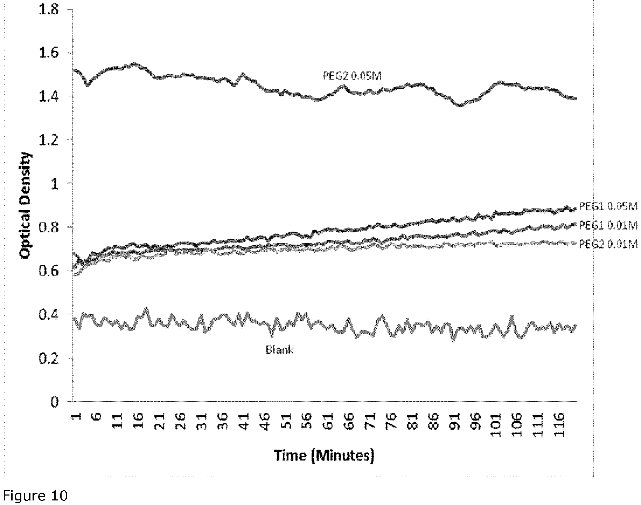

This has also been extended to a number of well-recognised general precipitation agents including ethanol, ammonium sulphate, and polyethylene glycol (PEG) (Figures 8, 9 and 10).

### Example 9 (further identification of precipitated protein)

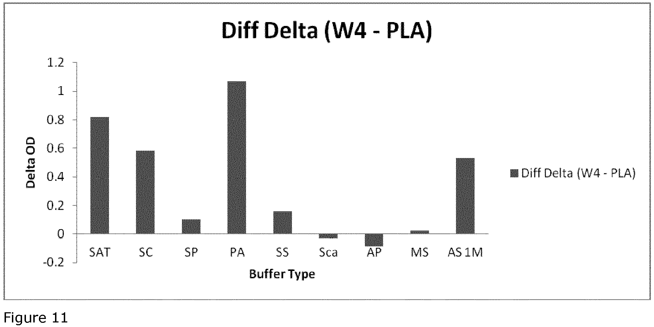

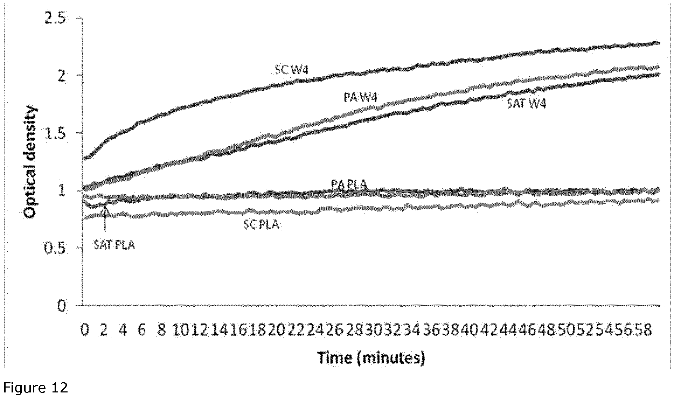

Using the afore mentioned buffers it was shown (Figures 11 and 12) that precipitation using these is differential when using equal amounts of sera from fish depending on disease state with sodium acetate, sodium citrate and potassium acetate appearing to result in the highest degree of precipitation.

### Mass spectroscopy of proteins

In total 36 protein spots of the W4pc pool from the resulting 2DE gel were excised and subjected to trypsin digestion before protein identification via electrospray ionisation (ESI) mass spectrometry on an Amazon ion trap MS/MS (Bruker Daltonics) as described in Braceland et al. (2013).

Chosen protein spots were excised manually by scalpel and placed in individual vials to be subjected to in-gel digestion for protein extraction prior to identification via mass spectrometry analysis. Gel pieces were washed with 100 mM NH4HCO3 for 30 min and then for a further hour with 100 mM NH4HCO3 in 50%(v/v) acetonitrile.

After each wash all solvent was discarded. Gel plugs were then dehydrated with 100% acetonitrile for 10 min prior to solvent being removed and dried completely by vacuum centrifugation. Dry gel pieces were then rehydrated with 10 µl trypsin at a concentration of 20 ng/µlin 25 mM NH4HCO3 (Cat No. V5111, Promega, Madison, WI, USA) and proteins allowed to digest overnight at 37 °C. This liquid was transferred to a fresh tube, and gel pieces washed for 10 min with 10 µl of 50% acetonitrile. This wash was pooled with the first extract, and the tryptic peptides dried to completion. Tryptic peptides were solubilized in 0.5% (v/v) formic acid and fractionated on a nanoflow uHPLC system (Thermo RSLCnano) before analysis by electrospray ionisation (ESI) mass spectrometry on an Amazon ion trap MS/MS (Bruker Daltonics).

Peptide separation was performed on a Pepmap C18 reversed phase column (LC Packings), using a 5-85% v/v acetonitrile gradient (in 0.5% v/v formic acid) run over 45 min. at a flow rate of 0.2µl/min. Mass spectrometric (MS) analysis was performed using a continuous duty cycle of survey MS scan followed by up to five MS/MS analyses of the most abundant peptides, choosing the most intense multiply-charged ions with dynamic exclusion for 120 seconds.

MS data were processed using Data Analysis software (Bruker) and the automated Matrix Science Mascot Daemon server (v2.1.06). Protein identifications were assigned using the Mascot search engine to interrogate protein sequences in the NCBI databases restricting the search to teleostei, allowing a mass tolerance of 0.4 Da for both MS and MS/MS analyses. In addition, the search consisted of a carbamidomethyl fixed modification and a variable oxidation.

Mass spectroscopy is a tool currently used to study proteins. Proteins are initially separated using gel electrophoresis as described above. Proteins of interest are excised and subjected to in-gel digestion for extraction prior to identification via mass spectrometry analysis.

Mass spectrometry is an analytical technique which separates components of a sample by their mass. A sample is vaporised into a gas and ionized. The resulting ions are accelerated and focussed into a beam. The ion beam passes through a magnetic field which bends the charged ions. Lighter components or ions with more ionic charge will deflect in the field more than heavier or less charged components. A detector counts the number of different deflections and the data can be plotted s a spectrum of different masses. Different proteins have different masses which can be compared against a database to identify the constituents of a sample. Mass spectrometry can be used to determine absolute and

relative protein quantities, and can identify and quantify thousands of proteins from complex samples.

Thirty-six spots were identified for mass spectrometry analysis from W4pc sera (Table 2). Analysis of these spots identified a number of tissue derived enzymes which due to the pathological tissue damage associated with PD are increased in sera (Table 2).

Table 2 shows the protein spots of interest at W4pc. Spots excised and their corresponding identities found via mass spectrometry, score, peptide matches and percent (%) sequence coverage. Score is related to the confidence of the database to identify a given protein. Identification of protein identity is carried out using an algorithm or score where confidence is given through matching peptides in a sample which make up part of a whole protein (= matches) the % sequence coverage is an indication of how identified peptides equate to the full length of protein. In general a score of over 70 indicates a high degree of confidence in the assumed protein identification.

Matches is concerned with the number of peptides in the protein matched with the number of known proteins available on the database and the % sequence coverage is the alignment of the identified peptides with amino acid sequences.

As can be seen from table 2 below, a number of intracellular enzymes were identified such as; three isoforms of creatine kinase, enolase, aldolase, glyceraldehyde 3-phosphate dehydrogenase, and pyruvate kinase. There is also an increase in apolipoprotein A1 (spot 23-25, 33, 34) complement C9 (spots 2-4) serotransferrin (spot 5, 25) cathepsin (spot 8) and haemoglobin (36). The small precipitate formed with W0pc pooled sera comprises mainly apolipoprotein and haemoglobin.

Indeed many of these enzymes, such as; creatine kinase (CK), enolase, aldolase, and glyceraldehyde-3-phosphate dehydrogenase (GAPDH) have previously been shown in Braceland et al. (2013) to be increased in serum concentration associated with pathological damage e.g. muscle tissues.

**Table 2. Table 2 shows the protein spots of interest at W4pc. Spots excised and their corresponding identities found via mass spectrometry, score, peptide matches and percent (%) sequence coverage.**

| Spot # | ID | Score | Matches | % Seq. Coverage |
|---|---|---|---|---|
| 1 | No sig. Matches | - | - | - |
| 2 | Complement component C9 (Q4QZ25) | 185 | 13 | 13 |
| 3 | Complement Component C9 (Q4QZ25) | 408 | 32 | 19 |
| 4 | Complement Component C9 (Q4QZ25) | 91 | 4 | 5 |
| 5 | Serotransferrin-1 (P80426) | 797 | 58 | 33 |
| 6 | Enolase 3-1 (B5DGQ6) | 112 | 7 | 14 |
| 7 | Enolase 3-2 (B5DGQ7) | 261 | 24 | 26 |
| | Enolase 3-1 (B5DGQ6) | 213 | 20 | 22 |
| | Cathespin M (Q70SU8) | 175 | 63 | 40 |
| 8 | Cathespin M (Q70SU8) | 150 | 29 | 30 |
| 9 | Fructose-bisphosphate aldolase (B5X0T0) | 309 | 20 | 25 |
| | Creatine Kinase-2 (B5DGP0) | 290 | 35 | 40 |
| | Creatine Kinase-3 (B5DGP2) | 257 | 34 | 36 |
| 10 | Pyruvate Kinase (C0H8V3) | 109 | 4 | 6 |
| 11 | Enolase 3-2 (B5DGQ7) | 767 | 79 | 41 |
| | Enolase 3-1 (B5DGQ6) | 635 | 71 | 39 |
| 12 | Enolase 3-1 (B5DGQ6) | 583 | 51 | 41 |
| 13 | Enolase 3-2 (B5DGQ7) | 596 | 50 | 29 |
| | Enolase 3-1 (B5DGQ6) | 547 | 41 | 27 |
| 14 | Aldolase a, fructose-bisphosphate 1 (B5DGM9) | 251 | 16 | 26 |
| 15 | Creatine Kinase-2 (B5DGP0) | 400 | 57 | 42 |
| | Creatine Kinase-3 (B5DGP2) | 340 | 53 | 42 |
| | Creatine Kinase (B5DGN9) | 263 | 45 | 31 |
| 16 | Creatine Kinase-2 (B5DGP0) | 462 | 63 | 44 |
| | Creatine Kinase-3 (B5DGP2) | 427 | 60 | 40 |
| | Creatine Kinase (B5DGN9) | 285 | 44 | 35 |
| 17 | Creatine Kinase-2 (B5DGP0) | 501 | 68 | 45 |
| | Creatine Kinase-3 (B5DGP2) | 452 | 64 | 45 |
| | Creatine Kinase (B5DGN9) | 356 | 56 | 40 |
| 18 | Creatine Kinase-2 (B5DGP0) | 426 | 67 | 39 |
| | Creatine Kinase-3 (B5DGP2) | 365 | 60 | 35 |
| | Creatine Kinase (B5DGN9) | 191 | 36 | 27 |
| 19 | Creatine Kinase-2 (B5DGP0) | 491 | 77 | 39 |
| | Creatine Kinase-3 (B5DGP2) | 415 | 64 | 35 |
| | Creatine Kinase (B5DGN9) | 181 | 44 | 27 |
| 20 | Aldolase a, fructose-bisphosphate 1 (B5DGM9) | 727 | 60 | 48 |
| 21 | Glyceraldehyde 3-phosphate dehydrogenase (042259) | 210 | 29 | 36 |
| 22 | Glyceraldehyde 3-phosphate dehydrogenase (042259) | 251 | 31 | 48 |
| 23 | Apolipoprotein A-I (P27007) | 773 | 129 | 55 |
| | Apolipoprotein A-I-2 (057524) | 264 | 55 | 23 |
| 24 | Apolipoprotein A-I (P27007) | 1970 | 241 | 76 |
| | Apolipoprotein A-I-2 (057524) | 350 | 66 | 30 |
| 25 | Apolipoprotein A-I (P27007) | 571 | 92 | 56 |
| | Serotransferrin-2 (P80429) | 182 | 9 | 10 |
| 26 | Apolipoprotein A-I (P27007) | 216 | 37 | 56 |
| 27 | Creatine Kinase-2 (B5DGP0) | 279 | 57 | 30 |
| | Creatine Kinase (B5DGN9) | 149 | 45 | 17 |
| 28 | Triosephosphate isomerise B (NP_001133174.1) | 652 | 60 | 40 |
| | Triosephosphate isomerase (B5DGL3) | 365 | 18 | 8 |
| 29 | Triosephosphate isomerise B (NP_001133174.1) | 471 | 33 | 40 |
| | Triosephosphate isomerase (B5DGL3) | 295 | 14 | 8 |
| 30 | Aldolase a, fructose-bisphosphate 1 (B5DGM9) | 349 | 30 | 30 |
| | Aldolase a, fructose-bisphosphate 2 (B5X0T0) | 332 | 26 | 28 |
| 31 | Triosephosphate isomerase (Q70I40) | 197 | 9 | 27 |
| 32 | Type-4 ice-structuring protein LS-12 | 664 | 33 | 54 |
| 33 | Apolipoprotein A-I (P27007) | 149 | 24 | 52 |
| | Apolipoprotein A-I-2 (057524) | 131 | 12 | 23 |
| 34 | Apolipoprotein A-I (P27007) | 214 | 52 | 39 |
| | Apolipoprotein A-I-2 (057524) | 154 | 16 | 46 |
| 35 | Creatine Kinase-3 (B5DGP0) | 112 | 13 | 19 |
| 36 | Hemoglobin subunit beta (Q91473) | 385 | 39 | 79 |
| | Alpha-globin 1 (A9YVA1) | 90 | 5 | 18 |

A clear difference in the precipitate was observed between W4pc and W0pc. The majority of spots identified that were present in W4pc precipitate but absent in W0pc. Creatine kinease, enolase, aldolase and glyceraldehydrate-3-phosphate dehydrogenase were found to precipitate in W4pc serum when combined with sodium acetate trihydrate buffer due to their increasing abundance associated with PD related pathological damage to tissues.

The development of lesions over time is illustrated in Figure 6, which shows mean lesion scores for heart, pancreas, white muscle and red muscle at each sampling point. Lesions developed in the pancreas from week 2 and were the slowest to recover, with a minority of tissue samples still not showing full recovery by week 12. The heart demonstrated a quick recovery, with a peak in lesion severity in fish sampled in week 4 and then a rapid recovery. The histopathological damage to red and white muscle was delayed with the peak damage occurring at 6 and 8 weeks respectively.

### References

Adams A & Thompson KD (2011) Development of Diagnostics for Aquaculture - Challenges and Opportunities. Aquaculture Research, 42, 93-102.
Asche Fr., Guttormsen A.G., Nielsen R. (2013) Future challenges for the maturing Norwegian salmon aquaculture industry: An analysis of total factor productivity change from 1996 to 2008. Aquaculture. 396-399, 43-50.
M. Braceland, R. Bickerdike, J. Tinsley, D. Cockerill, M.F. McLoughlin, D.A. Graham, , R.J. Burchmore, W. Weir, C. Wallace, P.D. Eckersall (2013) The serum proteome of Atlantic salmon, Salmo salar, during pancreas disease (PD) following infection with salmonid alphavirus subtype 3 (SAV3)J Proteomics, 94. 423-436.
Braceland M. M.F. McLoughlin, J. Tinsley, C. Wallace, D. Cockerill, M. McLaughlin, P.D. Eckersall Serum enolase: a non-destructive biomarker of white skeletal myopathy during pancreas disease (PD) in Atlantic salmon Salmo salar L. Journal of fish diseases (IN PRESS).
Centola et al., 2013: Centola, M., Cavet, G., Shen, Y., Ramanujan, S., Knowlton, N., Swan, K. A, Curtis, J. R. (2013). Development of a multi-biomarker disease activity test for rheumatoid arthritis. PloS One, 8(4), 1-6.
Eckersall PD, Bell R. Acute phase proteins: Biomarkers of infection and inflammation in veterinary medicine. Veterinary Journal. 185. 23-27.
Haluzova et al. (2010): Haluzova I., Modra H., Blahova., Marsalek P., Siroka Z., Groch., Svobodova. (2010) Effects of subchronic exposure to Spartakus (prochloraz) on common carp Cyprinus carpio. Neruro endocrino lett. 2, 105-113.
Jansen M.D., Wasmuth M.A., Olsen A.B., Gjerset B., Modahl I., Breck O., Haldorsen R.N., Hjelmeland R. Taksdal T. (2010) Pancreas disease (PD) in sea-reared Atlantic salmon, Salmo salar L., in Norway; a prospective, longitudinal study of disease development and agreement between diagnostic test results. Journal of Fish Diseases 33, 723-736.
Kibenge FSB, Godoy MG, Fast M, Workenhe S, Kibenge MJT, (2012). Countermeasures against viral diseases of farmed fish. Antiviral Research.
A.B. Kristoffersen, H. Viljugrein, R.T. Kongtorp, E. Brun, P.A. Jansen. Risk factors for pancreas disease (PD) outbreaks in farmed Atlantic salmon and rainbow trout in Norway during 2003-2007. Prev. Vet. Med., 90 (2009), pp. 127-136.
Larsson et al., 2012: Larsson T., Mørkøre T.,. Kolstad K, Østbye, S. Afanasyev T.-K., Krasnov A. (2012) Gene expression profiling of soft and firm Atlantic salmon fillet. PLoS ONE. 7, p. e39219.
Lerfall et al., 2011: Lerfall J., Larsson T., Birkeland S., Taksdal T., Dalgaard P., Afanasyev S., Bjerke M.T., Mørkøre T. Effect of pancreas disease (PD) on quality attributes of raw and smoked fillets of Atlantic salmon (Salmo salar L.) Aquaculture. 324-325, 209-217
Nielsen, 2002: Nielsen K., 2002. Diagnosis of brucellosis by serology. Veterinary Microbiology, 90, 447-459.
McLoughlin M.F., Graham D.A., Norris A., Matthews D.,Foyle L., Rowley H.M., Jewhurst H., MacPhee J. &Todd D. (2006) Virological, serological and histopathological evaluation of fish strain susceptibility to experimental infection with salmonid alphavirus. Diseases of Aquatic Organisms 72, 125-133.
Tacona A.G.T, Metian M. (2008) Global overview on the use of fish meal and fish oil in industrially compounded aquafeeds: Trends and future prospects 285. 146-158.
Yada et al. 2004: Yada T., Muto K., Azuma T., Ikuta K. (2004) Effects of prolactin and growth hormone on plasma levels of lysozyme and ceruloplasmin in rainbow trout, Comparative Biochemistry and Physiology Part C: Toxicology & Pharmacology. 139, 57-63.

## Claims

1. Diagnostic method for determining whether a fish is infected with a pathogen capable of causing pathological tissue damage in a fish wherein the method comprises the consecutive steps of:
i) mixing fish blood serum with an aqueous protein precipitation solution that causes increased protein precipitation when admixed with serum from tissue damaged fish compared to the corresponding protein precipitation observed in clinically healthy fish,
ii) determining whether the fish is infected by establishing:
iia) whether visible precipitate is formed, which in the affirmative is indicative of infection in the fish, or
iib) whether the resulting mixture has no visible precipitate, which in the affirmative is indicative of clinically healthy fish.

2. Diagnostic method for determining the stage of a pathogen causing disease resulting in pathological tissue damage in fish, the method comprising the consecutive steps of:
i) successively withdrawing blood samples from fish over a period of time (e.g. W0pc, W1pc, W2pc etc.)
ii) mixing the fish blood serum from the blood samples obtained under i) with an aqueous protein precipitation solution that causes increased protein precipitation when admixed with serum from tissue damaged fish compared to the corresponding protein precipitation observed in clinically healthy fish,
iii) determining the concentrations of the resulting protein precipitation samples from each of the mixtures obtained in step ii)
iv) comparing the concentrations of each of the protein precipitation samples and thereby determining the stage of the disease by establishing:
iva) whether the precipitation concentration of a sample (e.g. W2pc) is higher than the concentration of the immediately preceding sample (e.g. W1pc) which in the affirmative is indicative of progress of disease, or
ivb) whether the precipitation concentration of a sample (e.g. W2pc) is essentially equal to the concentration of the immediately preceding sample (e.g. W1pc) which in the affirmative is indicative of to steady state stage of the disease ivc) whether the precipitation concentration of a sample (e.g. W2pc) is lower than the concentration of the immediately preceding sample (e.g. W1pc) which in the affirmative is indicative of recovery from the disease.

3. Diagnostic method for determining the stage of a pathogen causing disease resulting in pathological tissue damage in fish, the method comprising the consecutive steps of:
i) successively withdrawing blood samples from fish over a period of time (e.g. W0pc, W1pc, W2pc etc.)
ii) mixing the fish blood serum from each of the blood samples obtained under i) with an aqueous protein precipitation solution that causes increased protein precipitation when admixed with serum from tissue damaged fish compared to the corresponding protein precipitation observed in clinically healthy fish,
iii) determining the optical density (level of turbidity) of the sample mixed with aqueous protein precipitation solution,
iv) determining the optical density (level of turbidity) in a sample mixed with aqueous protein precipitation solution (e.g. W2pc) and in the immediately preceding sample (e.g. W1pc)
v) optionally repeat step iv) with further successively obtained samples mixed with aqueous protein precipitation solution (e.g. W3pc, W4pc etc.),
vi) determining the change in optical density of the samples mixed with aqueous protein precipitation solution determined in step iv) and optionally in step v)
vii) thereby assessing the severity of the infectious disease as said severity is proportional with the degree of change in optical density (i.e. the higher the degree of change in optical density the more severe is the infectious disease).

4. Method according to any of claims 1-3, wherein the aqueous protein precipitation solution is chosen from solutions in water of one or more acids, bases, salts, organic compounds, inorganic compounds, ionic buffers or mixtures thereof.

5. Method according to claim 4, wherein the salt is chosen from ammonium sulphate, magnesium sulphate, ammonium phosphate, sodium carbonate, sodium sulphate, potassium acetate, sodium citrate, sodium phosphate, sodium acetate trihydrate or mixtures thereof.

6. Method according to claim 4, wherein the organic compound is chosen from methanol, ethanol, acetone or mixtures thereof.

7. Method according to claim 4, wherein the inorganic compound is polyethyleneglycol (PEG).

8. Method according to any of claims 1-7, wherein the fish is of the family Salmonidae such as Atlantic salmon (*Salmo salar L.*)*,* Adriatic trout (*Salmo obtusirostris),* Flathead trout (*Salmo platycephalus),* Marble trout (*Salmo marmoratus*), Ohrid trout *(Salmo letnica*), Sevan trout (*Salmo ischchan*)*,* Brown trout (*Salmo trutta*), also including fish from the genus *Salvelinus,* such as Arctic char (*Salvelinus alpinus),* also including fish from the genus Oncorhynchus, such as rainbow trout (*Oncorhynchus mykiss*), coho salmon (*Oncorhynchus kisutch*) and Chinook salmon (*Oncorhynchus tshawytscha*)*.*

9. Method according to any of claims 1-8, wherein the pathogen is chosen from the group consisting of Salmon α-virus, salmonid alphavirus, Salmon Pancreas Disease Virus (SPDV), salmonid alphavirus (SAV), Piscine Myocarditis Virus, Piscine reovirus (PRV), Atlantic Salmon Calcivirus (ASCV), Aquabirnaviruses, Infectious pancreatic necrosis virus (IPNv), ISA virus or viral hemorrhagic septicemia virus (VHSV).

10. Method according to any of claims 1-8, wherein the pathogen is chosen from the group consisting of *Aeromonas salmonicidia* subsp *salmonicidia, Vibrio salmonicidia, Vibrio anguillarum, Moritella viscosus, Piscirickettsia salmonis, Yersinia ruckeri* or *Flavobacterium psychrophilum.*

11. Method according to any of claims 1-8, wherein the pathogen is *Saprolegnia spp.*

12. Method according to any of claims 1-8, wherein the pathogen is chosen from the group consisting of *Neoparameabe peruans* or *Ichthyophthirius multifiliis.*

13. Method according to any of claims 1-8, wherein the pathological tissue damage is caused by one or more of the following diseases: Pancreas Disease (PD), Salmon Pancreas Disease (SPD), Heart and Skeletal Muscle Inflammation (HSMI), infectious pancreatic necrosis (IPN), Myopathy Syndrome (CMS), Infectious salmon anaemia (ISA), viral hemorrhagic septicemia (VHS), acute myopathy, immune reactions during chronic diseases caused by viruses, furunculosis, cold water vibriosis, vibriosis, winter ulcer disease, piscirickettsiosis, salmonid rickettsial septicemia (SRS), enteric redmouth (ERM), rainbow trout fry syndrome (RTFS), bacterial kidney disease (BKD), Saprolegniasis, Ameobic gill disease (AGD) or freshwater white spot disease.

14. Method according any of claims 1-13, wherein the determination of visible precipitate is detected by the naked eye.
